# EUROPEAN PATENT APPLICATION

(11) **EP 3 388 513 A1**
(43) Date of publication of application: **17.10.2018**
(21) Application number: 17166389.1
(22) Date of filing: 12.04.2017
(51) Int. Cl.: C12N 5/0775

(54) **METHOD OF OBTAINING DIFFERENTIATED CELLS FROM MUSCLE DERIVED PROGENITOR CELLS**

(71) Applicant: Revatis SA, 4000 Liege (BE)
(72) Inventor: SERTEYN, Didier, 4000 Liège (BE); CEUSTERS, Justine, 4000 Liège (BE)
(74) Representative: Bosch, Henry

(57) **Abstract**

The present invention relates to a method for preparing differentiated mammalian cells comprising collecting muscle microbiopsy, placing the sample in a cell culture medium, collecting and growing the cells and differentiating them in suitable differentiation medium.

## Description

The present invention relates to a new method for obtaining differentiated cells from muscle-derived progenitor cells (mdP-Cells).

Stem cells are characterized by self-renewal and multilineage differentiation. Many types of stem cells have ever been discovered but mesenchymal stem cells (MSC) have retained the most attention because of their ability to differentiate into mesodermal and non-mesodermal derivatives, their immunomodulatory potential, their broad availability and their contribution to maintain endogenous reserves of stem cells (Karantalis *et al.* 2015).

MSC are widely dispersed throughout the body but three sources are mainly used in clinical studies, they are bone marrow, adipose tissue and, to a lesser extent, umbilical cord (Lv *et al.* 2014; Karantalis *et al.* 2015). Bone marrow-derived mesenchymal stem cells are the most commonly used stem cells despite their painful sampling and their low production yield. Many other sources of stem cells have been investigated to address these shortcomings. Skeletal muscle contains cells able to self-renew and to differentiate into multiple lineages, not limited to myogenic differentiation as are satellite cells (Jankowski *et al.* 2002; Wu *et al.* 2010).

In 2006, for the sake of uniformity in view of the multitude of sources of MSC, the International Society for Cellular Therapy (ISCT) proposed minimal criteria to define human MSC: (1) MSC must be plastic-adherent in standard culture conditions; (2) MSC must express CD105, CD73 and CD90, and lack expression of CD45, CD34, CD14 or CD11b, CD79alpha or CD19 and HLA-DR surface molecules and (3) MSC must differentiate into osteoblasts, chondroblasts and adipocytes in vitro (Dominici *et al.* 2006).

Equine muscle-derived stem cells have been isolated from a muscular micro-biopsy using a patented method relying on explant culture followed by discontinuous Percoll density gradient to sort the stem cells-containing fractions with different densities (Serteyn and Ceusters 2015; WO2015/091210). This technique has allowed to reach approximately 60 millions stem cells in 6 weeks from a very small amount of muscle (15-20mg). Immunophenotyping with flow cytometry has shown that these cells were positive for CD105, CD90 and CD44 whereas they were negative for CD45 and MHC-II. Equine muscle-derived stem cells have proven their pluripotency by successfully differentiating into osteocytes, chondrocytes, adipocytes, (keratinocytes), hepatocytes, cardiomyocytes and endothelial cells. Furthermore, these cells have shown immunomodulatory properties. Therefore, these muscle-derived stem cells represent a population of mesenchymal stem cells (mdMSC) that can be abundantly and readily achieved in a micro-invasive manner, offering a good alternative to bone marrow-derived mesenchymal stem cells.

Stem cells represent a valuable tool for in vitro studies. In fact, once the differentiation protocol established, they enable to work in a very standardized manner and to create highly reproducible cellular models, replacing less suited models based on immortalized cells, cells derived from cadavers or from an other species than the targeted one (Pittenger et al. 2013 MSC book). Moreover, stem cells offer the opportunity to provide lots of different cell types from a single individual and thus from a single sampling, which constitutes an invaluable tool for research conducted on cell types whose sampling is dangerous or even unsafe. Furthermore, stem cells meet the need of creating individual therapeutic approaches which are of primary importance to assess efficacy and/or toxicity of different treatments on one or more cell types of the patient in question. Until now, the scientific literature obtains differentiated cells from mesenchymal stem cells originating from bone marrow, adipose tissue, blood, dental pulp, umbilical blood cord, ... after a phase of separation, culture and expansion.

It has now been found that differentiated cells may be obtained from a muscle microbiopsy without any prior separation technique to isolate different mensenchymal stem cell populations.

According to the invention, the method for preparing differentiated mammalian cells comprises (i) collecting a muscle microbiopsy sample from a mammalian individual, (ii) placing the collected muscle sample of step (i) in a suitable culture medium, (iii) growing the cells emerging from said microbiopsy, (iv) separating the said microbiopsy and growing cells when a sufficient amount of growing cells is present around the muscle sample, (v) continuing the growing of the cells to near confluency and (vi) differentiating the cells from step (v) in a suitable differentiation medium.

According to a preferred embodiment of the invention, the said muscle microbiopsy is taken from skeletal muscle tissue.

The separation of the microbiopsy muscle sample and the cells that have emerged from it and grown around it may be effected by removing the tissue sample, thus leaving the cells in the relevant culture medium for further growing.

The culture medium advantageously comprises DMEM/F12 with about 20% fetal bovine serum, about 5 ml penicillin (1000 U/ml)-streptomycin (10000 µg/ml), about 2.5 ml amphotericin B (250 µg/ml) and about 5 ml HEPES. Further examples are CTS (commercial denomination) and Therapeak (commercial denomination) culture medium.. The skilled person may find other culture mediums appropriate as well and will be able to adapt the culture medium to the requirements of the process, making use of his common knowledge, experience and skills.

The mammal may be selected from the group comprising domestic and farm animals, zoo animals, sport animals, pet animals, companion animals and experimental animals, including but not limited to mice, rats, hamsters, rabbits, dogs, cats, guinea pigs, cattle, cows, sheep, horses, pigs and primates, for example monkeys and apes, but also from humans.

Advantageously, the microbiopsy is obtained from skeletal muscle tissue, such as from muscles from the neck, shoulder, chest, back, tail, limbs, hindlimb, forelimb, hindquarters, hindleg etc. In the case of a mammal like a horse the microbiopsy is preferably obtained from triceps brachii muscle tissue, more preferably taken from the long head of the triceps brachii. In the case of a human, the microbiopsy may be obtained from the deltoid muscle. The person skilled in the art will be able to select a suitable source without undue burden.

In the case of a horse, for instance, the micro-biopsy may be collected at a depth of about 5 cm in the long head of the triceps brachii and may contain about 10 to about 30 mg, preferably more than about 12 mg or more than about 15 mg and/or less than about 25 mg or less than about 20 mg of tissue. In the case of a human, the microbiopsy may contain from about 10 to about 20 mg of tissue. Clearly, though, depending on the mammal and skeletal muscle tissue source, the skilled person will be able to adapt the depth of microbiopsy and quantity of tissue sample to be extracted, on the basis of his general knowledge and/or by routine experimentation without undue burden.

The isolated muscle-derived progenitor cells may be differentiated into adipocytes, osteocytes, chondrocytes, myogenic cells, hematopoetic cells, endothelial cells, neural cells, cardiac cells, or hepatocytes by culturing them in an adequate adipogenic, osteogenic, chondrogenic, myogenic, hematopoetic, endothelial, neuronal, cardial, or hepatocytic differentiation medium, respectively. The relevant differentiation mediums are known to the skilled person who will be able to select appropriate mediums and culture conditions on the basis of his common knowledge, experience and skills, without undue burden.

The terms "muscle-derived progenitor cells" or "mdP-Cells" as used herein are understood to mean all the cells that have come out of the micro-biopsy by explant culture without separating the different stem cell-containing fractions, e.g. by using discontinuous Percoll density gradient.

Cell differentiation is understood to mean a process where a cell changes from one cell type to a generally more specialized type. This process may be triggered by appropriate medium controlling gene expression. The terms "differentiated cell(s)" as used herein are understood to mean specialized cell types such as adipocytes, osteocytes, chondrocytes, myogenic cells, hematopoietic cells, endothelial cells, neural cells, cardiac cells, or hepatocytes, which are more specialized than the progenitor cells or stem cells they are derived from.

The term "stem cell" refers generally to an unspecialised or relatively less specialised and proliferation-competent cell, which is capable of self-renewal, i.e., can proliferate without differentiation, and which or the progeny of which can give rise to at least one relatively more specialised cell type. The term encompasses stem cells capable of substantially unlimited self-renewal, i.e., wherein the progeny of a stem cell or at least part thereof substantially retains the unspecialised or relatively less specialised phenotype, the differentiation potential, and the proliferation capacity of the mother stem cell, as well as stem cells which display limited self-renewal, i.e., wherein the capacity of the progeny or part thereof for further proliferation and/or differentiation is demonstrably reduced compared to the mother cell. By means of example and not limitation, a stem cell may give rise to descendants that can differentiate along one or more lineages to produce increasingly relatively more specialised cells, wherein such descendants and/or increasingly relatively more specialised cells may themselves be stem cells as defined herein, or even to produce terminally differentiated cells, i.e., fully specialised cells, which may be post-mitotic.

The term "mesenchymal stem cell" or "MSC" as used herein refers to a mammalian adult, mesoderm-derived stem cell that is capable of generating cells of mesenchymal lineages, typically cells of two, preferably of three or more mesenchymal lineages, e.g., osteocytic (bone), chondrocytic (cartilage), myocytic (muscle), tendonocytic (tendon), fibroblastic (connective tissue), adipocytic (fat) and stromogenic (marrow stroma) lineage. Commonly, but without limitation, a cell may be considered MSC if it is capable of forming cells of each of the adipocytic, chondrocytic and osteocytic lineages, using standard, art-accepted differentiation conditions and cellular phenotype evaluation methods, e.g., as described in Pittenger et al. 1999 (Science 284: 143-7) or Barberi et al.,2005 (PLoS Med 2: e161), and U̅sas et al., 2011. The term MSC also encompasses the progeny of MSC, e.g., progeny obtained by *in vitro* or ex *vivo* propagation of MSC obtained from a biological sample of a subject.

The ISCT determined precisely the qualities cells must possess to be defined as mesenchymal stem cells (MSCs) as follows: the cells must be plastic-adherent, positive for the markers CD73, CD90 and CD105, negative for the markers CD14 (or CD11 b), CD34, CD45, CD79a (or CD19) and MHC-II, and must exhibit the ability to differentiate into cells of mesodermal origin such as osteoblasts, chondroblasts and adipocytes (Dominici et al., 2006). The use of other MSC markers such as CD29 or CD44 was also reported (Pittenger et al., 1999). The mammalian MSC cells as used in accordance with the present invention hence are defined in that they express or co-express (i.e., are positive for) at least the mesenchymal marker CD105, and preferably also one or more of the following markers: CD44 and CD90. The mammalian MSC cells of the present invention are also defined in that they may express or co-express (i.e., are positive for) one or more of the following microRNAs: miR-128, miR-133B, miR-218 or miR-802. The mammalian MSC cells of the present invention are also defined in that they do not express miR-656.

The terms microRNA, miRNA, miR or eca-miR are used herein interchangeably, and refer to 19-25 nucleotides mature non-coding RNAs or precursors thereof, or fragments thereof, derived from endogenous genes of living organisms such as animals. Mature microRNAs are processed from longer hairpin-like precursors termed pre-microRNAs (pre-miRs) having a length of approximately 75 nucleotides.

Where a cell is said to be positive for a particular marker or microRNA, this means that a skilled person will conclude the presence or evidence of a distinct signal, e.g., antibody-detectable or detection by reverse transcription polymerase chain reaction, for that marker or microRNA when carrying out the appropriate measurement, compared to suitable controls. Where the method allows for quantitative assessment of the marker or microRNA, positive cells generate a signal that is significantly different from and higher or stronger than the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

The expression of cell-specific markers can be detected using any suitable immunological technique known in the art, such as immuno-cytochemistry or affinity adsorption, Western blot analysis, FACS, ELISA, etc., or by any suitable biochemical assay of enzyme activity, or by any suitable technique of measuring the quantity of the marker mRNA, e.g., Northern blot, semi-quantitative or quantitative RT-PCR, etc.

The expression of microRNAs may be determined, for example, with an assay for global gene expression (e.g. using a microarray assay for microRNAs expression profiling analysis, a ready-to-use microRNA qPCR plate or RNA sequencing) or by specific detection assays, for example, but not limited to, quantitative PCR, quantitative reverse-transcription (real-time) PCR (qRT-PCR), locked nucleic acid (LNA) real-time PCR, or northern blotting. In particular, the measurement of the expression of a microRNA may be carried out with an oligonucleotide probe specific for the detection of said microRNA. Said oligonucleotide probe may bind directly and specifically to the microRNA, or may specifically reverse transcribe said microRNA. Alternatively, said oligonucleotide probe may bind a cDNA obtained from said microRNA. Said oligonucleotide probe may also amplify a cDNA obtained form said microRNA.

The terms "growing" and "culturing"as used herein are understood to mean that the cells multiply up to a certain level of confluency.

The terms "sufficient amount of cells around the microbiopsy" means that sufficient cells have grown on and/or in the proximity of the muscle sample such that they can be separated from the muscle sample to further grow.

The method according to the invention provides an easy source of differentiated cells, including but not limited to adipocytes, osteocytes, chondrocytes, myogenic cells, hematopoietic cells, endothelial cells, neural cells, cardiac cells, and hepatocytes, for in vitro research, including for example toxicity assays, organ-on-a-chip based experiments, drug development and personalized medical treatment development. Latter may comprise assessing the likelihood of a positive response by the patient by evaluating the level of the positive response of the said mammalian differentiated cells.

The present invention offers an effective and promising alternative to the methods already described in the literature and provides the possibility of being carried out on living mammals due to the minimally invasive character of the progenitor cell collection. As is known, muscle-derived cells are a mixture of subpopulations from different lineages and different developmental stages, including stem cells. Cell culture may be initiated with a simple method: the muscular microbiopsies may be used as explants and progenitor cells appear spontaneously in due time. By doing so, the number of manipulations is reduced, avoiding potential sources of contamination, and no external growth factors need to be added, since the growth factors that are naturally secreted by the muscle microbiopsy (the explant) are sufficient.

The present invention is particularly suitable for autologous cell usage, implying more reliable results that are less depending on genetic particularities or anomalies.

The present invention provides also methods of treatment by administering the differentiated cells to subjects in need thereof, which phrase includes subjects (mammals) that would benefit from treatment of a given condition. Such subjects may include, without limitation, those that have been diagnosed with said condition, those prone to develop said condition and/or those in whom said condition is to be prevented. The differentiated cells may be used in the treatment of one or more of the following disorders: osteocyte differentiated cells for the treatment of, osteoporosis, fracture, and failure to heal in a mammalian subject; chondrocyte differentiated cells for the treatment of desmitis, osteochondrosis, arthritis tendonitis, laminitis, inflammation of the tendons and ligaments; myogenic cells and cardiac cells for the treatment of cardiac disorders, such as improvement of cardiac function, reconstruction of infarcted cardiac tissue; hematopoeitic differentiated cells for the treatment of autoimmune diseases and cancer of the blood and bone marrow, such as multiple myeloma and leukemia; endothelial cells for the treatment of endothelial dysfunction; neural cells for the treatment of degenerative neural diseases, Parkinson's disease, Huntington's disease, multiple sclerosis; hepatocyte differentiated cells for the treatment of liver dysfunction and cancer.

The terms "treat" or "treatment" encompass both the therapeutic treatment of an already developed disorder, such as the therapy of an already developed myo-arthro-skeletal disorder, as well as prophylactic or preventative measures, wherein the aim is to prevent or lessen the chances of incidence of an undesired affliction, such as to prevent the chances of contraction and progression of a myo-arthro-skeletal disorder such as but not limited to: desmitis, osteochondrosis, arthritis, osteoporosis, tendonitis, inflammation of the tendons and ligaments, fracture, and failure to heal. Beneficial or desired clinical results of such a treatment may include, without limitation, alleviation of one or more symptoms or one or more biological markers, diminishment of extent of disease, stabilised *(i.e.,* not worsening) state of disease, delay or slowing of disease progression, amelioration or palliation of the disease state, and the like. "Treatment" can also mean prolonging survival as compared to expected survival if not receiving treatment.

The term "prophylactically effective amount" refers to an amount of the veterinary or pharmaceutical composition according to the invention that inhibits or delays in a subject the onset of a disorder as being sought by a researcher or veterinarian. The term "therapeutically effective amount" as used herein, refers to an amount of the veterinary or pharmaceutical composition according to the invention that elicits the biological or medicinal response in a subject that is being sought by a researcher, or veterinarian, which may include *inter alia* alleviation of the symptoms of the disease or disorder being treated. Methods are known in the art for determining therapeutically and prophylactically effective doses.

The treatment may employ autologous (*i.e.,* cells derived from the subject to be treated), allogeneic (*i.e.,* cells derived from subject(s) other than the subject to be treated, but belonging to the same species) or xenogeneic (*i.e.,* cells derived from subject(s) belonging to species other than the subject to be treated) differentiated cells or their respective cell populations as defined herein.

The veterinary or pharmaceutical compositions will typically comprise the differentiated cells or cell populations as obtained in accordance with the invention as the active ingredient, and one or more pharmaceutically acceptable carrier/excipient. As used herein, "carrier" or "excipient" includes any and all solvents, diluents, buffers (such as, *e.g*., neutral buffered saline or phosphate buffered saline), solubilisers, colloids, dispersion media, vehicles, fillers, chelating agents (such as, *e.g*., EDTA or glutathione), amino acids (such as, *e.g*., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for veterinary or pharmaceutical active substances is well known in the art. Such materials should be non-toxic and should not interfere with the activity of the cells. For veterinary use, the cells could also be formulated in, or administered as, a feed supplement.

The precise nature of the carrier or excipient or other material will depend on the route of administration. For example, the composition may be in the form of a parenterally acceptable aqueous solution. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Such veterinary or pharmaceutical compositions may contain further components ensuring the viability of the (differentiated) cells or cell populations therein. For example, the compositions may comprise a suitable buffer system (e.g., phosphate or carbonate buffer system) to achieve desirable pH, more usually near neutral pH, and may comprise sufficient salt to ensure isoosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, e.g., albumin, which may increase the viability of the cells.

The veterinary or pharmaceutical compositions may comprise further components useful in the repair or regeneration of the relevant tissue or organ to be treated or functionality thereof. As an example, the veterinary or pharmaceutical compositions may comprise components useful in the repair of bone wounds and defects. For example, such components may include without limitation bone morphogenetic proteins, bone matrix (e.g., bone matrix produced in vitro by cells of the invention or by other methods), hydroxyapatite/tricalcium phosphate particles (HA/TCP), gelatine, poly-lactic acid, poly-lactic glycolic acid, hyaluronic acid, chitosan, poly-L-lysine, and collagen. For example, the osteoblastic cells may be combined with demineralised bone matrix (DBM) or other matrices to make the composite osteogenic (bone forming in it own right) as well as osteo-inductive.

The veterinary or pharmaceutical composition can further include or be co-administered with a complementary bioactive factor such as a bone morphogenic protein, such as BMP-2, BMP-7 or BMP-4, or any other growth factor. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a granular ceramic, or a biopolymer such as gelatine, collagen, osteonectin, fibrinogen, or osteocalcin. Porous matrices can be synthesized according to standard techniques (e.g., Mikos et al., Biomaterials 14: 323, 1993).

The veterinary or pharmaceutical composition can further include or be co-administered with a complementary disinfecting, aseptic, or microorganism destroying agent such as a bactericidal, antibacterial, antibiotic, or antifungal and/or an anti-inflammatory agent in order to avoid complications due to infection and or inflammation at the site of introduction or administration of the differentiated cells.

The differentiated cells or cell populations can be administered in a manner that permits them to graft or migrate to the intended tissue site and reconstitute or regenerate the functionally deficient area. Administration of the composition will depend on the myo-arthro-skeletal site being repaired. For example, the differentiated cells or cell populations can be administrated either directly in the lesions (such as for example in tendon or ligament), or in the synovial joints (such as for example the tendinous or articular synovials).

For example, osteogenesis can be facilitated in concordance with a surgical procedure to remodel tissue or insert a split, or a prosthetic device. In other circumstances, invasive surgery will not be required, and the composition can be administered by injection, such as ultra-sound guided injection, or using a guidable endoscope.

In another embodiment, the differentiated cells or cell populations of the invention may be transferred to and/or cultured on suitable substrates to provide for implants. The substrate on which the cells can be applied on part at least of the surface and cultured can be a metal, such as titanium, cobalt/chromium alloy or stainless steel, a bioactive surface such as a calcium phosphate, polymer surfaces such as polyethylene, and the like. Although less preferred, siliceous material such as glass ceramics, can also be used as a substrate. Most preferred are metals, such as titanium, and calcium phosphates, even though calcium phosphate is not an indispensable component of the substrate. The substrate may be porous or non-porous. The substrate may be biodegradable or bioabsorbable.

For example, differentiated cells obtained in accordance with the invention can be transferred onto three-dimensional solid supports in order to cause them to multiply and/or continue the differentiation process by incubating the solid support in a liquid nutrient medium of the invention, if necessary. Cells can be transferred onto a three-dimensional solid support, e.g. by impregnating said support with a liquid suspension containing said cells. The impregnated supports obtained in this way can be implanted in a subject. Such impregnated supports can also be re-cultured by immersing them in a liquid culture medium, prior to being finally implanted.

The three-dimensional solid support needs to be biocompatible so as to enable it to be implanted in a subject. It can be of any suitable shape such as a cylinder, a sphere, a plate, or a part of arbitrary shape. Of the materials suitable for the biocompatible three-dimensional solid support, particular mention can be made of calcium carbonate, and in particular aragonite, specifically in the form of coral skeleton, porous ceramics based on alumina, on zirconia, on tricalcium phosphate, and/or hydroxyapatite, imitation coral skeleton obtained by hydrothermal exchange enabling calcium carbonate to be transformed into hydroxyapatite, or else apatite-wollastonite glass ceramics, bioactive glass ceramics such as Bioglass(TM) glasses.

In the present description, term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

For general methods relating to the invention, reference is made to well-known textbooks, including, e.g., "Molecular Cloning: A Laboratory Manual, 2nd Ed." (Sambrook et al., 1989), Animal Cell Culture (R. I. Freshney, ed., 1987), the series Methods in Enzymology (Academic Press), Gene Transfer Vectors for Mammalian Cells (J. M. Miller & M. P. Calos, eds., 1987); "Current Protocols in Molecular Biology and Short Protocols in Molecular Biology, 3rd Ed." (F. M. Ausubel et al., eds., 1987 & 1995); Recombinant DNA Methodology II (R. Wu ed., Academic Press 1995), incorporated by reference herein

For further elaboration of general techniques useful in the practice of this invention, the practitioner can refer to standard textbooks and reviews in cell biology, tissue culture, and embryology. Included are "Teratocarcinomas and embryonic stem cells: A practical approach" (E. J. Robertson, ed., IRL Press Ltd. 1987); "Guide to Techniques in Mouse Development" (P. M. Wasserman et al. eds., Academic Press 1993); "Embryonic Stem Cell Differentiation in Vitro" (M. V. Wiles, Meth. Enzymol. 225:900, 1993); "Properties and uses of Embryonic Stem Cells: Prospects for Application to Human Biology and Gene Therapy" (P. D. Rathjen et al., al., 1993). Differentiation of stem cells is reviewed, e.g., in Robertson. 1997. Meth Cell Biol 75: 173; and Pedersen. 1998. Reprod Fertil Dev 10: 31, and U̅sas et al., 2011, incorporated by reference herein.

General techniques in cell culture and media collection are outlined in Large Scale Mammalian Cell Culture (Hu et al. 1997. Curr Opin Biotechnol 8: 148); Serum-free Media (K. Kitano. 1991. Biotechnology 17: 73); Large Scale Mammalian Cell Culture (Curr Opin Biotechnol 2: 375, 1991), Culture of animal cells: A manual of basic technique, Freshney, R.I. et al. 2005, 5th Edition, Wiley, New York, incorporated by reference herein.

Nucleic and amino acid sequence data for marker proteins listed in this disclosure are generally known and can be obtained from public databases such as, among others, from the NIH "Protein Reviews on the Web" database (http://mpr.nci.nih.gov/prow/), the NIH "Entrez Gene" database (http://www.ncbi.nlm.nih.gov/sites/entrez?db=gene) or the Uniprot/Swissprot database (http://www.expasy.org/). Suitable detection reagents and methods for said markers can be designed either on the basis of such sequence information or, more commonly, are available commercially (e.g., labelled monoclonal antibody reagents).

The term "CD105" encompasses the antigen known as CD105, or its synonyms such as endoglin. CD105 is a membrane glycoprotein located on cell surfaces and is a known mesenchymal stem cell marker. As an example, the partial amino acid sequence of the equine CD105 antigen can be found in the Genbank database under accession number AGW16345.1.

The term "CD90" encompasses the antigen CD90, or its synonyms such as Thy-1 membrane glycoprotein. As an example, the amino acid sequence of the equine CD90 antigen can be found in the Genbank database under accession number ACG61223.1.

The term "CD44" encompasses the antigen generally known as CD44, or its synonyms such as Extracellular matrix receptor III, GP90 lymphocyte homing/adhesion receptor, HUTCH-I, Hermes antigen, Hyaluronate receptor, or Phagocytic glycoprotein 1. As an example, the amino acid sequence of the equine CD44 antigen can be found in the Genbank database under accession number CAA47331.1.

Exemplary commercially available antibody reagents for detection of said MSC markers include *inter alia* monoclonal antibodies anti-CD105-RPE (ABD Serotec), anti-CD44-APC (BD Pharmigen), and anti-CD90 (VMDR). Alternative antibodies that are specifically binding to CD105, CD44, or CD90 can be identified by the person skilled in the art.

MicroRNAs listed in this disclosure are generally known and can be obtained from public databases such as, among others, the miRBase database (http://www.mirbase.org). The term "miR-128" encompasses the microRNA known as miR-128 or its precursor. As an example, the nucleotide sequence of the equine miR-128 can be found in the miRBase database under accession number MI0012821. The term "miR-133B" encompasses the microRNA known as miR-133B or its precursor. As an example, the nucleotide sequence of the equine miR-133B can be found in the miRBase database under accession number MI0012844. The term "miR-656" encompasses the microRNA known as miR-656 or its precursor. As an example, the nucleotide sequence of the equine miR-656 can be found in the miRBase database under accession number MI0012915. The skilled person is well aware that microRNAs may be referred to by different names, or synonyms.

The term "cell population" generally refers to a grouping of cells. A cell population may consist of or may comprise at least a fraction of cells of a common type, or having characteristics in common. Such characteristics may include, without limitation, morphological characteristics, potential for differentiation (e.g., pluripotent, multipotent, unipotent, etc.; e.g., if multipotent or unipotent, ability to differentiate towards specific cell types), or the presence and/or level of one, two, three or more cell-associated markers, e.g., surface antigens. Such characteristics may thus define a cell population or a fraction thereof. Preferably, such a cell population is mesenchymal stem cell population, more preferably a substantially homogenous population of mesenchymal stem cells.

The expression "density gradient centrifugation" encompasses all types of cell-separation techniques or products encompassing the density-based separation of cells. Non-limiting examples can be density gradient centrifugation in a gradient of sucrose polymer, or colloidal silica. Non-limiting examples of commercially available gradients are: percoll (colloidal silica coated with polyvinylpyrrolidone or silane), ficoll (high molecular weight sucrose-polymers), Ficoll-Paque (Ficoll plus sodium diatrizoate and edetate calcium disodium), buoyant density solution (BDS, comprising colloidal silica), lymphoprep (sodium diatrizoate and polysaccharide), etc.

Live cells having a desired expression profile are allowed to bind with reagents (most commonly immunological reagents such as, e.g., monoclonal antibodies) specific for the respective markers, wherein said reagents are in turn modified (e.g., by a fluorophore, or by immobilisation on magnetic particles or another type of stationary phase), such as to facilitate for selection or capture of cells bound by said reagents from cells not so bound. For general guidance on these methods, refer *inter alia* to Flow Cytometry and Cell Sorting, 2nd ed., by Andreas Radbruch (ed.), Springer 1999 (ISBN 3540656308); In Living Color: Protocols in Flow Cytometry and Cell Sorting, 1st ed., by RA Diamond and S Demaggio (eds.), Springer 2000 (ISBN 3540651497); Flow Cytometry Protocols (Methods in Molecular Biology), 2nd ed., by TS Hawley and RG Hawley (eds.), Humana Press 2004 (ISBN 1588292355); Affinity Separations: A Practical Approach, P Matejtschuk (ed.), Oxford University Press, 1997 (ISBN 0199635501); and Dainiak et al. 2007. Adv Biochem Eng Biotechnol 106: 1 - 18.

The expression "suitable culture medium" encompasses all cell-culturing media that support the survival and/or growth of the cells, mesenchymal stem cells (MSCs) or mesenchymal stem cell populations. Non-limiting examples are: DF20, DMEM-Ham's F12, DMEM, Alpha-MEM etc., typically supplemented with at least antibiotics and fetal bovine serum (FBS), and optionally with antifungal agents and buffers. As an example only, the following culture medium has been used in the examples: DF20 medium comprising: DMEM/F12 with about 20% fetal bovine serum, about 5ml penicillin (1000U/ml)-streptomycin (10000µg/ml), about 2.5ml amphotericin B (250µg/ml) and about 5ml HEPES. Other examples are CTS (commercial denomination) and Therapeak (commercial denomination) culture medium.

For differentiation into e.g. adipocytes, osteocytes and chondrocytes, the progenitor cells where cultured in an adequate "differentiation medium". Said differentiation medium can for example be: for adipogenic differentiation: NH AdipoDiff Medium (Miltenyi Biotec); for chondrogenic differentiation: chondrocyte differentiation medium (NH ChondroDiff Medium; Miltenyi Biotec); for osteogenic differentiation: osteogenic medium (NH OsteoDiff Medium; Miltenyi Biotec). The media listed herein are merely shown as exemplary media, but the skilled person will be able to use any other commercial or specifically developed differentiation medium. Other examples of suitable differentiation media for other cells such as myogenic cells, hematopoetic cells, endothelial cells, neural cells, cardiac cells, or hepatocytes can be done by culturing the progenitor cells in an adequate myogenic, hematopoetic, endothelial, neuronal, cardial, or hepatocytic differentiation medium respectively, examples of which can e.g. be found in Usas et al., 2011.

Appropriate ways of "detaching", "dispersing", "dissociating" or "disassociating" cells are generally known in the art and may be used in the present invention. These involve, e.g., treatment with proteolytic enzymes, chelation of bivalent ions, mechanical disintegration, or combinations of any of the above. Preferably, said cell dissociation may involve enzymatic digestion, favourably using trypsin (e.g., as described above), optionally in combination with chelation of bivalent ions, favourably using EDTA (e.g., as described above), and/or mechanical dissociation of the so-treated cells. The latter may involve, e.g., repeated passing of the cells through a small bore pipette (e.g., a 1000µl micropipette tip) and/or pipetting out a stream of a suspension containing the cells against a solid surface (e.g., against the wall of the culture vessel).

The present invention will be described in more details with reference to the preparation of differentiated chondrocytes or osteocytes from muscle-derived progenitor cells.

### Example

Obtaining differentiated chondrocytes or osteocytes from the differentiation of mdP-Cells avoids invasive and painful bone biopsy in a patient potentially suffering from bone disease.

### Material and Methods

### Sampling of equine skeletal muscle

Equine skeletal muscle has been collected from a healthy donor at the Equine Clinic of the University of Liege. The experimental protocol has been reviewed and approved by the Animal Ethics Committee of the University of Liege (agreement n°1162). The muscular micro-biopsy has been performed as reported by Serteyn and Ceusters (2015). The micro-biopsy specimen has been achieved form the triceps brachii muscle (long head, at the intersection of a vertical line extending from the tricipital crest and a line between the scapulo- and radio-humeral joints) on standing horse without sedation. The sample has been collected with a 14-gauge micro-biopsy needle and micro-biopsy pistol. The sampling site has been shaved (1cm² is enough), aseptically prepared and 2ml lidocaïne 2% (Xylocaïne, AstraZeneca, Sweden) have been injected subcutaneously. Skin has been incised with the tip of a scalpel blade No 15 and the micro-biopsy needle has been advanced at a depth of 5cm. Skin incision has not been closed as deemed unnecessary and the whole procedure has been completed within 15 minutes. Immediately after collection, the sample (15-20mg of muscle tissue) has been placed in 10ml of culture medium composed of DMEM/F12 (Dulbecco's modified Eagle's medium/Ham's F12 (1:1 mix), 15mM HEPES, with L-glutamine; Lonza, Verviers, Belgium; BE12-719F) supplemented with 20% fetal bovine serum (FBS; Gibco; 10500-064), penicillin (100UI/ml)-streptomycin (100µg/ml) (Lonza, Verviers, Belgium; DE17-602E) and amphotericin B (1.25µg/ml) (Lonza, Verviers, Belgium; 17-836E). The sample has been kept in culture medium at 4°C and the time limit before using it has not exceeded 72 hours.

### Isolation of mdP-Cells using explant culture

The micro-biopsy specimen must be handled very cautiously to avoid damaging muscle tissue. Culture preparation has been performed under aseptic conditions using sterile equipment and working under a laminar flow hood. The sample has been rinsed twice in 5ml phosphate buffered saline (PBS) (DPBS, Dulbecco's Phophate Buffered Saline 0.0095M (PO₄), without Ca, without Mg; Lonza, Verviers, Belgium; BE17-512F) preheated to 37°C before being carefully dissected in PBS trying to remove as much as possible non-muscular tissue. Afterwards, remaining muscular tissue has been divided into very small pieces of about 1 mm side length. Pieces have been distributed at a rate of 6 or 7 per well into 4 wells of a 6-well plate. Next, culture medium preheated to 37°C has been added cautiously, drop by drop, until the amount is considered sufficient (about 1ml), pieces just need to be covered. Indeed, the lack of culture medium does not prevent the drying of the explants and does not provide sufficient nutrient to emerging cells while excess prevents adhesion of the explants that hence remain floating. The 2 wells remaining empty have been filled with 1ml PBS to prevent drying out of wells containing the explants. In the end, the 6-well plate has been incubated at 37°C under controlled atmosphere (5% CO₂ and 21% O₂). Wells containing explants have been daily-monitored and culture medium has been added when necessary. Culture medium has not been changed in order to keep secreted growth factors within the wells. Wells containing PBS have been replenished when necessary.

When the newly appeared cells have formed a halo around the pieces of muscle tissue (about 10 days), the explants have been removed to prevent their necrosis and cells have been given additional time to reach 80% confluence (about 10 days).

### Osteogenic or chondrogenic differentiation of directly isolated mdP-Cells

When cells reached 80% confluence, culture medium has been completely removed and osteogenic or chondrogenic differentiation has been conducted. Cells have been maintained during 7 to 21 days in osteogenic or chondrogenic differentiation medium. Each well has been filled with 2 ml differentiation medium, medium has been totally changed once a week. Cells have been incubated at 37°C under controlled atmosphere (5% CO₂ and 21% O₂). After 7 days, osteogenic differentiated cells were washed in PBS and fixed in 70% ethanol at room temperature for 5 min followed by several washes in H2O. Cells were then stained in 40mM Alizarin Red (Sigma) pH 4.2 for 15 min at room temperature, rinsed in H2O, and then air dried. Red staining was examined by light microscopy. After 21 days, chondrogenic differentiated cells were fixed in 70% ethanol at room temperature for 5 min followed by several washes in H2O. Cells were then stained in Alcian Blue, rinsed in H2O and then air dried. Blue staining was examined by light microscopy.

The method of the present invention avoids painful biopsy in a patient potentially suffering from bone disease and further enables the production of increased amounts of chondrogenic or osteogenic cells as compared to prior art techniques.

## Claims

1. A method for preparing differentiated mammalian cells, comprising (i) collecting a muscle microbiopsy sample from a mammalian individual, (ii) placing the collected muscle sample of step (i) in a suitable culture medium, (iii) growing the cells emerging from said microbiopsy, (iv) separating the microbiopsy and growing cells when a sufficient amount of growing cells is present around the muscle sample, (v) continuing the growing of the cells to near confluency, and (vi) differentiating the cells obtained at (v) in a suitable differentiation medium.

2. Method according to claim 1 wherein the said muscle microbiopsy is taken from skeletal muscle tissue.

3. Method according to claim 1 or 2 wherein the micro-biopsy contains about 10 to about 30 mg, preferably more than about 12 mg, more preferably more than about 15 mg and/or less than about 25 mg, preferably less than about 20 mg.

4. Method according to any preceding claim wherein differentiation is made into adipocytes, osteocytes, chondrocytes, myogenic cells, hematopoetic cells, endothelial cells, neural cells, cardiac cells, or hepatocytes by culturing them in an adequate adipogenic, osteogenic, chondrogenic, myogenic, hematopoetic, endothelial, neuronal, cardial, or hepatocytic differentiation medium, respectively.

5. Differentiated adipocytes, osteocytes, chondrocytes, myogenic cells, hematopoetic cells, endothelial cells, neural cells, cardiac cells, or hepatocytes as obtained by the method of any of caims 1 to 4.

6. A pharmaceutical or veterinary composition comprising differentiated adipocytes, osteocytes, chondrocytes, myogenic cells, hematopoetic cells, endothelial cells, neural cells, cardiac cells, or hepatocytes of claim 5.

7. A substrate comprising differentiated cells of claim 5 on part at least of its surface.

8. A method of treatment of mammalian subjects comprising administering differentiated cells of claim 5 to subjects in need thereof.

9. The method of treatment of claim 8 comprising the treatment of one or more of the following disorders: desmitis, osteochondrosis, arthritis, osteoporosis, tendonitis, laminitis, inflammation of the tendons and ligaments, fracture, and failure to heal in a mammalian subject.
